Europäisches Patentamt

(19) European Patent Office

Office européen des brevets

(11) Publication number: **0 235 153 B1**

(12) **EUROPEAN PATENT SPECIFICATION**

(45) Date of publication of patent specification :
12.06.91 Bulletin 91/24

(51) Int. Cl.⁵: **G01N 33/53, C12M 1/40**

(21) Application number: **86900212.1**

(22) Date of filing: **18.12.85**

(86) International application number:
**PCT/GB85/00590**

(87) International publication number:
**WO 86/03837 03.07.86 Gazette 86/14**

(54) METHOD FOR BIOCHEMICAL ASSAY.

(30) Priority: **19.12.84 GB 8432069**

(43) Date of publication of application:
**09.09.87 Bulletin 87/37**

(45) Publication of the grant of the patent:
**12.06.91 Bulletin 91/24**

(84) Designated Contracting States:
**BE CH DE FR GB IT LI NL SE**

(56) References cited:
**EP-A- 60 123**
**EP-A- 142 301**
**US-A- 3 984 766**
**US-A- 4 156 180**
**US-A- 4 334 880**

(56) References cited:
**ANALYTICAL CHEMISTRY, vol. 52, no. 11, Sep 1980, Easton, PA (US); W.J.BLAEDEL et al.: "Reagentless enzyme electrodes for ethanol, lactate, and malate", pp. 1691-1697**
**BIOTECHNOLOGY AND BIOENGINEERING, vol. 25, no. 3, March 1983, New York, NY (US); J.P.KERVENEZ et al.: "Determination of substrate concentrations by a computerized enzyme electrode", pp. 845, 852-854**

(73) Proprietor: **IQ (BIO) LIMITED**
**Downham House Downham's Lane Milton Road**
**Cambridge CB4 1XG (GB)**

(72) Inventor: **STANLEY, Christopher, John**
**161 The Spinney,**
**Bar Hill, Cambridge (GB)**

(74) Representative: **Raynor, John et al**
**W.H. Beck, Greener & Co 7 Stone Buildings Lincoln's Inn**
**London WC2A 3SZ (GB)**

## Description

This invention relates to methods and apparatus for biochemical assay, and in particular to immunoassays and nucleic and probe assays in which at least one reagent is linked to an enzyme. Immunoassays make use of biochemical substances which have specific reactivity with other substances, for example antigens and antibodies. Immunoassay techniques are increasingly used in medicine for the detection of a wide range of substances, for example hormones, for purposes as diverse as the diagnosis of cancer, to the detection of oestrus in cattle. In a nucleic acid probe assay, a particular nucleic acid sequence in the DNA or RNA extracted from bacterial plan or animal cells may be detected by the use of a complimentary probe containing a known sequence of nucleotide bases.

Early biochemical assays were immunoassays based mainly on the incorporation in one of the components of the reaction of a radioisotope, or a fluorescent group as a label, so that the presence of a particular reaction product can be determined qualitatively and quantitatively.

Both of these techniques have disadvantages associated with them. Both require sensitive and expensive equipment. In addition, there are practical difficulties in the extent to which radioisotopes can be incorporated in antigens and antibodies, and the products may have short shelf lives. The use of fluorescent groups results in an assay which is difficult to standardise because of the background fluorescence of biological material so that quantitative results cannot be obtained easily.

The use of enzymes as labels in immunoassay has been a recent development and has offered the advantages of long shelf life, safe reagents and assay end points that can be read using readily available optical equipment. Conventional enzyme immunoassay has not, until recently offered an improvement in sensitivity over radio-immunoassay.

In DNA probe assays, the DNA probe is conventionally labelled, for example, with a radioactive atom such as phosphorus, directly with an enzyme such as alkaline phosphatase or indirectly with biotin incorporated into the probe and the subsequent addition of an enzyme conjugate, for example an avidin-alkaline phosphatase conjugate. The hybridisation of the labelled probe with the target sequence in the nucleic acid can be used to immobilise an enzyme to a surface, in much the same way as in an immunoassay. This enzyme can be detected by its generation of a coloured product or, as disclosed in International Patent Application PCT/GB84/00432 International by using an electroactive enzyme such as glucose oxidase which can be detected electrochemically.

Various techniques have recently been proposed for example in EP-A-0037036, EP-A-0019606, EP-A-0058635 and EP-A-0060123 for improving the sensitivity of enzyme assays, by the use of so-called "biochemical amplifiers".

In existing biochemical amplified systems, an enzyme-based immunoassay generates a trigger substance for a cyclic series of reactions, which can produce a much more substantial change than the conventional direct assay method. To date however, such amplified systems have been restricted to biochemical amplifiers which produce a colour change, which is measured optically.

In practice, these enzyme immunoassays are generally carried out by using a test plate having a large number of test wells, normally 96, and an optically clear bottom plate. Upon completion of the immunoassay, the test plate is inserted into a measuring instrument and an optical beam can be passed through each of the test wells in turn, so as to provide a reading of change in optical density for the various test wells. Each immunoassay is normally carried out in duplicate (ie. in at least two test wells), and in addition, various blanks and standards are needed in order to calibrate the results obtained. Because the optical measurement on each well takes a finite tine, and the plate has to be physically moved between each measurement, the scanning of the various test wells for even a single determination can take a substantial length of time, and because of the limited dynamic range of optical instruments it also makes it very difficult to obtain during the same test run accurate results for test solutions where the concentration of the test substance varies widely.

EP-A-0150999 (cited un der Art. 64 (3) EPC) discloses an imnunoassay method in which an enzyme label which catalyses a redox reaction, e.g. glucose oxidase, is bound to a stationary support in an immunoassay, and the products of the redox reaction which it catalyses are determined, indirectly via a ferrocene mediator, electrochemically. EP-A-0150999 is concerned solely with assays which bind an enzyme label which itself catalyses a redox reaction, the product of which may be determined, directly or via a mediator at an electrode surface. The sensitivity of the assays disclosed is low, and generally not sufficient to enable a practical assay to be constructed which is sufficiently sensitive to detect the low levels of analytes which are frequently encountered.

We have now found that if an enzyme immunoassay is carried out in which the bound enzyme label is not an enzyme that can catalyse a redox reaction directly, but instead catalyses a reaction, typically a hydrolytic reaction, which results in the production of a trigger substance for a cyclic chemical reaction or series of reactions, which generates a further product capable of taking part in an electrochemical redox reaction at an electrode, an electrochemical immunoassay can be produced which is of substantially enhanced sensitivity. The term "product" used above is intended to include a product of a cyclic reaction which is continuously produced and consumed during operation of the cycle.

The cyclic chemical reaction will in general itself be a redox cycle, although it may be a non-redox cycle, one product of which is capable either of reacting further at an electrode or of producing a further product which subsequently takes part in electrochemical oxidation or reduction at an electrode.

In such redox cycles one of the products of the cycle (for example NADH, in an $NAD^+$/NADH cycle or quinone in a catechol/quinone cycle) is capable of being, respectively, oxidised or reduced, either at the electrode itself, to produce a determinable electrochemical change, or via the active site of a suitable enzyme, or else in solution, to produce a change of the oxidation state of a mediator, which can then be determined electrochemically at the electrode. When the cyclic reaction is an $NAD^+$/NADH cycle, the NADH produced may be determined directly at a suitable electrode, for example a graphite electrode, doped with a suitable modifier, e.g. thionine, or benzoquinone. In an alternative and preferred embodiment, a soluble mediator or combination of mediators is employed, such as ferrocene, hexacyanoferrate, or phenazinium methyl sulphate (PMS).

When a chemical mediator is employed, a metal electrode may be utilised, for example a gold or platinum electrode.

Determination of the redox reaction at the electrode may be made by standard voltammetric techniques.

The immunoassay may be carried out so as to immobilise the enzyme label on any convenient surface, for example a wall of a test cell, or, as disclosed in EP-A-0150999, on the surface of magnetic particles. Alternatively, the enzyme label may be immobilised directly on the surface of an electrode.

It is particularly advantageous in an immunoassay to assemble together a plurality of test cells, each having electrodes connected to the tracks of a printed circuit board. This makes it possible to monitor simultaneously the progress of a large number of enzyme reactions, representing separate immunoassays, at relatively short time intervals, for example using a computer.

Accordingly, the determination is preferably carried out in a biochemical test cell assembly comprising at least one test cell for containing a test solution, a pair of electrodes in the test cell, and a biochemical ligand bound to a surface in the cell, the said ligand being adapted to bind specifically with a corresponding antiligand, thereby to immobilise on the said surface an enzyme label capable of being detected, indirectly, by its ability to trigger a further chemical reaction employing a redox cycle with an electrical effect on the said electrodes.

As indicated above, the assembly preferably includes a plurality, for example at least eight, test cells, each being connected to a track of a printed circuit board, which forms a part of the assembly. The printed circuit board preferably also incorporates a multi-way connector, to enable the simultaneous connection of measuring equipment to the various pairs of electrodes in the assembly. The measuring equipment may take the form of a micro-computer, including a micro-processor, which is adapted to read repeatedly a sequence of values from the electrode pairs, for example using a single analogue-to-digital converter and sequencing switch, and store the values associated with each reading, so as to provide a profile of the change in the said electrical value, for each of the electrode pairs, over a period of time.

The surface to which the ligand is bound may be one of the electrode surfaces, so that the antiligand to which the enzyme label is attached is conveniently situated close to the respective electrode, in order to facilitate electron transfer between the electrode surface and the substance which takes part in the electrochemical redox reaction.

When a biochemical amplifier is used (i.e. when the enzyme label catalyses a reaction which results in the production of a trigger substance for a cyclic chemical reaction, as described above), the ligand need not necessarily be bound to the electrode surface as the enzyme label may be measured indirectly by its triggering effect on the components of the biochemical "amplifier" which may be freely diffusing or some or all of which may be immobilised to the electrode surface to enhance electron transfer.

In a further embodiment of the invention, one electrode of the or each pair may be attached to a removable cover portion for the test cell. Thus, in an alternative aspect of the invention, there is provided a cover for a test cell assembly, comprising a plurality of pairs of electrodes mounted on and projecting from the cover at least one of the electrodes of each pair preferably having a biochemical ligand bound to its surface.

The method of the invention is equally suited for use in DNA probe assays. In a DNA probe assay in accordance with the invention the label which is utilised is the said enzyme, for example a phosphatase or a hydrolase, which does not itself catalyse a redox reaction, but catalyses a reaction which produces a trigger substance as indicated above. It is particularly advantageous in this case for the DNA or RNA hybridisation assay to be carried out in a nitrocellulose or nylon membrane or filter which is in direct contact with a pair of flat platinum electrodes bonded to the lower surface of the matrix. In this way the activity of the biochemical amplifier can be detected by the simple addition of the appropriate reagents to the filter and the application of a suitable potential to the electrodes.

A number of the particular embodiments of the invention will now be described, and are illustrated with reference to the accompanying drawings, in which :

Figure 1 is a schematic representation of a reaction scheme, in accordance with the invention,

Figure 2a to 2e are schematic representations of alternative reaction schemes,

Figure 3 is a schematic representation of the operation of the lipoamide dehydrogenase active site in a redox cycle involving NADH and an electrode,

Figure 4 is a schematic diagram of a test cell assembly according to the invention,

Figure 5 is a schematic diagram of a computer system adapted for use with the test cell assembly of Figure 4, and

Figures 6, 7 and 8 are experimental plots obtained in immunoassays.

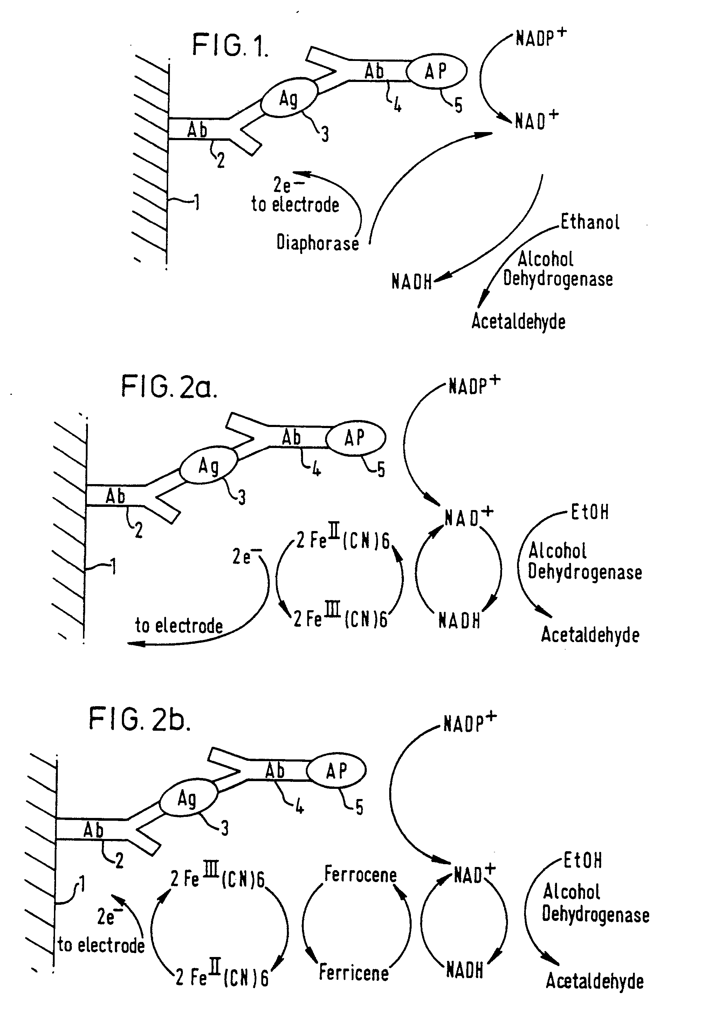

In the embodiment of the invention illustrated schematically in Figure 1, a surface 1 within a test cell which may be for example the wall of the test cell, a magnetic particle within the test cell, or the surface of an electrode, is prepared by binding an antibody 2, preferably a monoclonal antibody, to its surface. An antigen 3 to be determined is prepared in appropriate concentration, and caused to react selectively with the antibody. A second monoclonal antibody 4, having an enzyme 5, for example alkaline phosphatase, conjugated thereto is there caused to react with the antigen 3, according to the well known "sandwich" immunoassay technique.

The method of the invention is equally applicable to non-sandwich type assays, for example to so-called "competition" assays in which the antigen competes with an antigen-enzyme conjugate for immobilised antibody. Competition assays are particularly suitable for assaying small antigens and haptens. The electrochemical detection of the bound enzyme label is the same whatever the specific mechansims of the assay.

The amount of alkaline phosphatase linked to the surface 1 is proportional to the amount of antigen 3 in the sample, and can be determined electrically, as follows. The support, with bound alkaline phosphatase, is washed, and a solution is added containing nicotinamide adenine dinucleotide phosphate ($NADP^+$).

The $NADP^+$ provides a substrate for the alkaline phosphatase, which catalyses the production of nicotinamide adenine dinucleotide ($NAD^+$) by hydrolysis of a phosphate ester chemical bond. This $NAD^+$ serves as a trigger substance for a cyclic reaction (a so-called "biochemical amplifier"), resulting in the oxidation of a substrate in excess such as ethanol to acetaldehyde and the reduction of $NAD^+$ to NADH, catalysed by an $NAD^+$ specific dehydrogenase such as alcohol dehydrogenase. The NADH so formed is reoxidised by the active site of the enzyme diaphorase which is itself subsequently oxidised electrolytically at the electrode surface. This results in a measurable current, the magnitude of which is dependent upon the amount of $NAD^+$ produced by the enzyme label in the immunoassay. The great specificity of the enzymes alcohol dehydrogenase and diaphorase means that the substrate for alkaline phosphatase, $NADP^+$, is unlikely to enter the redox cycle.

In the example given in Figure 1 the electrode will accept electrons directly from the active site of the enzyme diaphorase. In an alternative embodiment the electrode in suitably modified form will accept electrons from NADH without the use of the enzyme diaphorase.

In a system such as this, it is not essential that the antibody 2 should be bound directly to the electrode surface, and the surface 1 can represent any suitable surface within the test solution, for example a wall of the test cell.

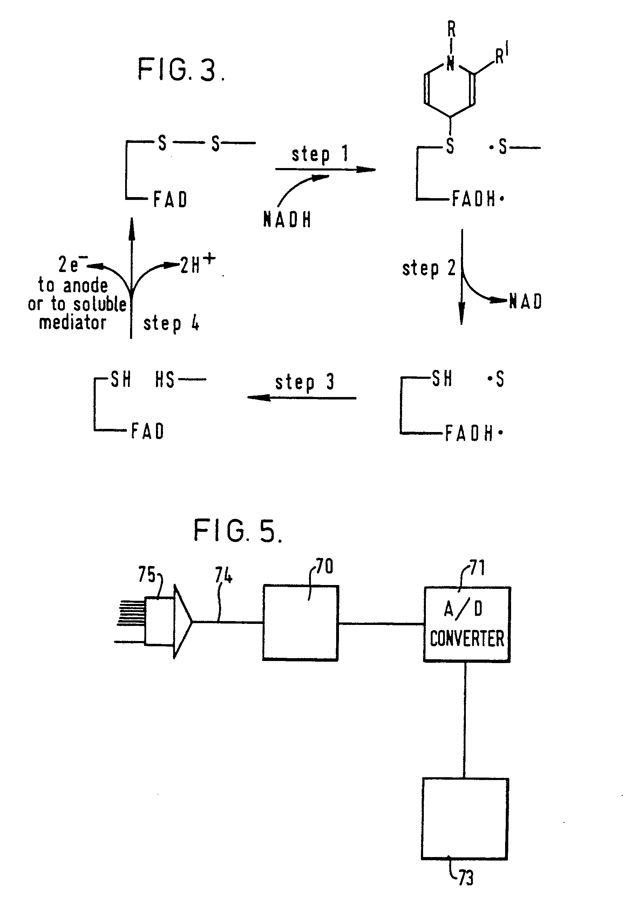

Figure 3 illustrates in more detail the reaction scheme for a redox cycle involving NADH, the active site of diaphorase (known systematically as lipoamide dehydrogenase) shown schematically as a disulphide bridge and a molecule of FAD, and an electrode surface. NADH first binds to one of the sulphur atoms as shown in step one of Figure 3, to'form 2 free radicals. $NAD^+$ then departs from the active site (step 2), and a rearrangement occurs (step 3) to reoxidise the FAD and reduce the remaining sulphur. The active site is then fully reoxidised by contact with an electrode or mediator in step 4 in which the electrons are donated to the electrode or mediator, and the disulphide bridge is reformed.

The $EO^1$ value for the $NADH/NAD^+$ redox system is −320 mV while that for the $FADH^2/FAD$ system in the lipoamide dehydrogenase active site is close to OmV. Electron acceptors such as ferricyanide ($EO^1$ = +418 mV) or ferrocene derivatives ($EO^1$ = +300 to +650 mV) or an electrode at a suitable potential will re-oxidise the diaphorase active site.

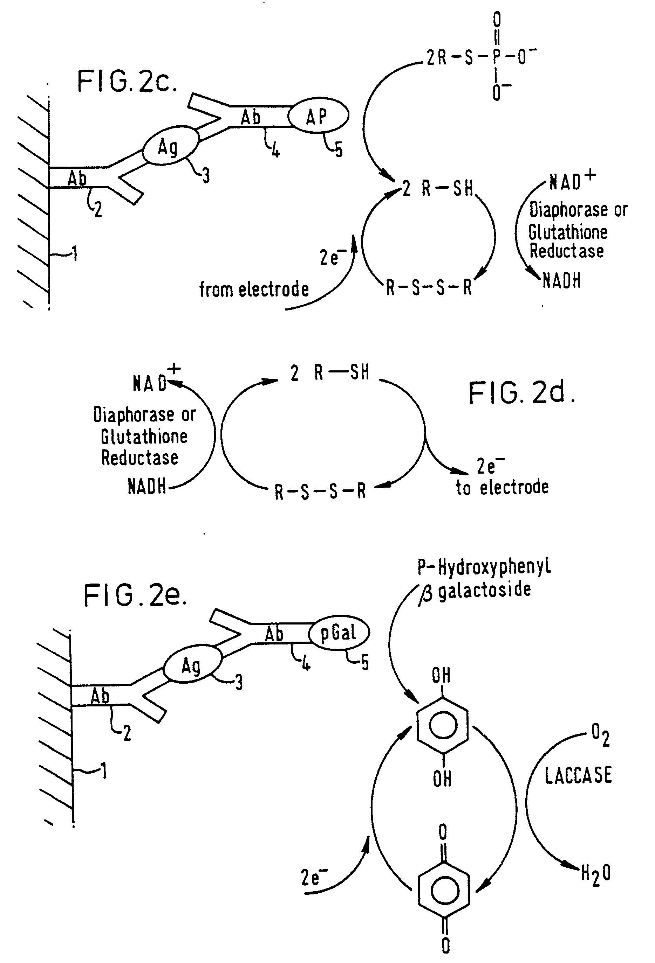

Figure 2a illustrates a similar reaction scheme to that of Figure 1, but in which the NADH produced in a cyclic reaction is used to reduce a mediator, the ferricyanide ion, to ferrocyanide, a process catalysed by the active site of diaphorase. The ferrocyanide in turn is oxidised by a platinum working electrode at a potential of +450 mV, to produce a current. Because the amount of ferrocyanide produced is dependent upon the amount of $NAD^+$ introduced into the cyclic reaction of the biochemical amplifier, which is in turn dependent upon the amount of alkaline phosphatase 5 bound to surface 1, the steady state current produced at the electrode is dependent directly upon the amount of antigen 3 bound in the immunoassay.

A further alternative scheme is illustrated in Figure 2b, in which an additional mediator, ferrocene is interposed between the $NAD^+/NADH$ cycle, and the ferricyanide/ferrocyanide cycle. In this case, NADH produced by the cyclic reaction of NAD$^+$ reduces ferricene to ferrocene, catalysed by diaphorase, which in turn reduces ferricyanide to ferrocyanide. Again, ferrocyanide is oxidised at a platinum working electrode at a potential of +450 mV, to produce a current which is measured.

There are a number of alternative chemical means to produce NAD$^+$ from precursors that are inactive in the biochemical redox amplification cycles described above. One alternative to the alkaline phosphatase reaction employs NAD$^+$ glycohydrolase as the enzyme label. This enzyme will produce NAD$^+$ from an analogue, NAD$^+$ dihydroxyacetone by a transfer reaction driven by an excess of nicotinamide.

Figures 2c and 2d illustrate alternative reaction schemes. In the example of Figure 2c, bound alkaline phosphatase 5 is used to hydrolyse a thiophosphate ester, to generate a free thiol (RSH). The free thiol serves as the trigger substance for a cyclic reaction, in which the thiol is converted to the disulphide RSSR and back again. This reaction can be caused to take place under the influence of an excess of NAD$^+$, catalysed by a suitable enzyme. When the thiol is lipoic acid, the enzyme may be diaphorase, or when the thiol is glutathione, the enzyme may be glutathione reductase.

In Figure 2c the thiol is regenerated by electrochemical reduction of the disulphide compound at a platinum working electrode (a cathode) at a suitable potential depending on the thiol compound chosen, but without effect on the NAD$^+$/NADH system (EO$^1$ −320 mV). An alternative redox cycle employing electrochemical oxidation of the thiol compound is shown in Figure 2d. Here the platinum working electrode (an anode) will accept electrons from R-SH, the disulphide is reduced by an excess of NADH via diphorase or glutathione reductase.

In the reaction scheme illustrated as Figure 2e the enzyme label in the immunoassay is β-galactosidase. The enzyme catalyses the hydrolysis of p-hydroxyphenyl β-galactoside generating 1,4 benzoquinol or catechol. This is the trigger molecule for a biochemical amplifier employing the enzyme laccase. Catechol is oxidised to 1,4 benzoquinone at the expense of oxygen, a platinum working electrode (a cathode) is used to reduce the quinone to quinol (EO$^1$ = +0,28 V) and the current is measured.

Varisous alternative redox cycles are possible in the method of the invention. FAD (flavin adenine dinucleotide) is a component of the diaphorase active site (Figure 3). It is also used by many redox enzymes. In the case of glucose oxidase the apoenzyme can be prepared which is electrochemically inactive. The generation of FAD by the enzyme label in an immunoassay from a precursor such as a galactoside results in the activation of glucose oxidase which then operates as a biochemical amplifier with glucose in excess (where the redox cycle is FAD/FADH$_2$ in the active site of glucose oxidase). EP-A-0 150 999 discloses a method measuring electrochemically the activity of glucose oxidase using the soluble mediator ferrocene monocarboxylic acid and a graphite electrode. In a further example the co-factor PQQ (pyrrolo-quinoline quinone) is used by certain redox enzymes such as methanol dehydrogenase. The generation of PQQ from a precursor such as a phosphate by the enzyme label in the immunoassay results in the activation of methanol dehydrogenase and the redox cycle PQQ/PQQH$_2$ driven by an excess of methanol can reduce an appropriate electrode using a suitable mediator.

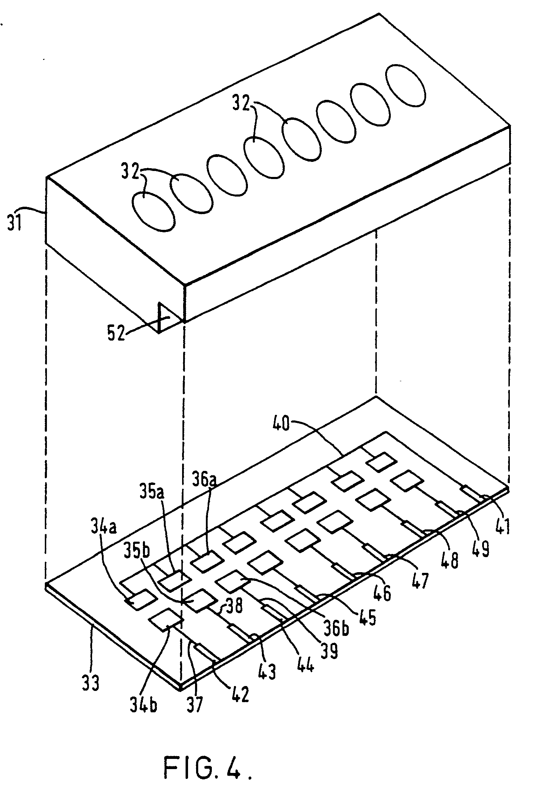

Figure 4 is a schematic diagram of a test cell assembly according to the invention. The assembly comprises a polystyrene block 31, approximately 10 mm thick, having 8 through holes 32 therein. The block 31 is secured by means of an epoxy adhesive to a printed circuit board 33. The printed circuit board 33 carries eight pairs of electrodes, 34a, 35a, 36a, etc. and 34b, 35b, 36b, etc. bonded to its surface. The electrodes 34a, 35a, 36a etc. are joined by a common copper track 40 to a terminal 41, forming a common, or ground terminal. The electrodes 34b, 35b, 36b etc. are each joined by a respective copper track 37, 38, 39, etc. to a respective terminal 42, 43, 44 etc.

Thus, the terminals 41 to 49 constitute a conventional "edge" connector as used in conventional circuit board technology. The copper tracks 39 to 40 may preferably be formed on the underside of the printed circuit board 33, so as not to interfere with the glueing of the block 31 to the circuit board 33. A portion 52 of the block 31 is cut away so as to facilitate the use of the edge of the circuit board 33 as an edge connector. Thus, when the block 31 is glued in position on the plate 33, a test cell assembly is formed comprising a plurality of test cells defined by the holes 32, and the bottom plate 33, each having within it a pair of electrodes, which can be accessed via the edge connector constituted by terminals 41 to 49.

The 8 × 1 array of test cells illustrated is simply illustrative, and in a practical embodiment, an array of, perhaps, 12 × 8 might be used, to provide 96 test cells, each having two electrodes therein.

The test cell surfaces may be coated with a ligand material, for example a monoclonal antibody, by any known means, for example by simply introducing an appropriate solution of the antibody into the test cell, allowing it to stand, and decanting the liquid.

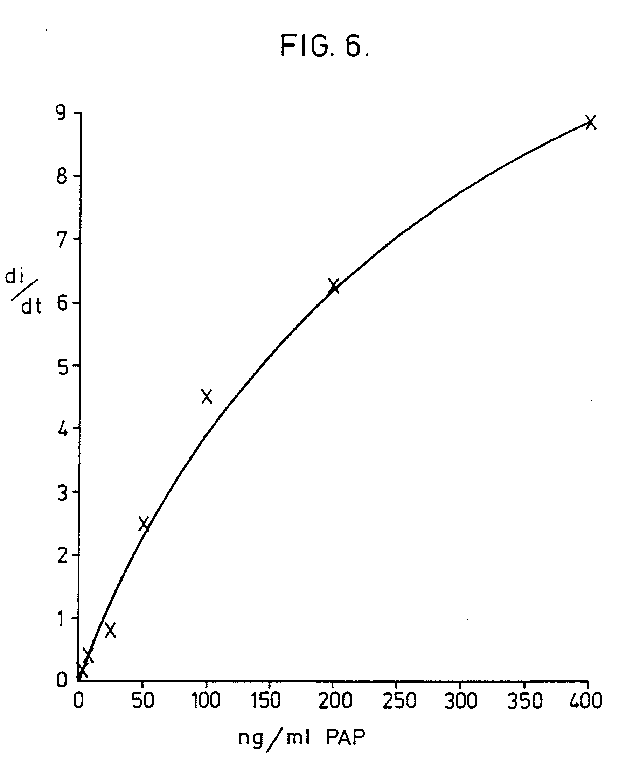

Figure 5 illustrates a simplified computer arrangement for carrying out an electrochemical immunoassay using the test cell assembly of Figure 4. A 9-way connector 75 adapted to connect with the terminals 41 to 49 of the assembly Figure 4 is connected via a 9-way ribbon cable 74 to a software-driven sequence switch 70. The sequencing switch 70 connects each of the electrodes 34b, 35b, 36b etc. in turn to the input of an analogue-to-digital converter 71, in turn connected to a micro computer 73. The micro computer 73 is adapted to sample each of the cells 32 at regular intervals, for example every second, and to store the current values associated therewith. Once stored the values can be accessed as desired to provide kinetic measurements of the progress of the enzyme assays.

In an alternative embodiment, the electrodes may be formed by a plurality of pins formed on a printed circuit board, and adapted to project downwardly into the wells of a conventional optical microtitre plate, for example a plate such as that produced by NUNC. Such a board may be provided with an edge connector, as described above.

The electrode may be formed of a metal, or metal-treated plastic, carbon, or other conducting or semi-conducting material. In particular, for the direct oxidation of NADH, a suitable electrode may be produced by treating graphite with benzoquinone, or, much more preferably, thionine.

A number of particular embodiments of the invention are described in the following examples.

Example 1

An electrochemical immunoassay for human prostatic acid phosphatase was carried out in accordance with the following method, using the principle outlined in Figure 2a.

The wells of a 96-well polystyrene plate (NUNC Immunoplate I) were coated with a monoclonal antibody to human PAP (prostatic acid phosphatase) by diluting the antibody to a concentration of 5 µg/ml in 1.0 mM-sodium carbonate buffer pH 9.0 and pipetting 100 ul of the solution into each well. The plate was incubated for 16 hours at 37°C and excess antibody was removed by washing with the carbonate buffer. Then, to each well was added 75 µl of a solution containing 50 ng/ml of a conjugate of a second monoclonal antibody and alkaline phosphatase (made according to methods given in "Enzyme Immunoassay" Ishikawa, Kawai and Miyai 1981, Igaku-Shoin, Tokyo) in 100 mM-triethanolamine buffer pH 7.5 containing 6% (w/v) bovine serum albumin, 1 mM-magnesium chloride, 0.05% (v/v) Triton X705 and 0.1% (w/v) sodium azide, followed immediately by 25 µl of human serum containing known amounts of human PAP. The plate was incubated for 2 hours at 22°C during which time a sandwich of immobilised antibody, PAP and conjugate formed on the surface of the well. Excess conjugate and serum was removed by washing the wells with a solution of 0.1% Triton®-X705, 25 mM-Tris buffer pH 8.0 and 200 mM ammonium sulphate. To each well was added 200 µl of a 0.2 mM solution of NADP+ (nicotinamide adenine dinucleotide phosphate) in 50 mM-diethanolamine buffer pH 9.5. After 20 minutes at 22°C the enzyme reaction was stopped by the addition of 20 µl of 0.10 M-sodium phosphate pH 7.0. A total of 12 replicate wells at each PAP concentration were pooled in order to provide sufficient material for the electrochemical cell.

The concentration of NAD+ formed by the action of alkaline phosphatase on NADP+ was then measured electrochemically. The cell had a volume of 2 ml and contained platinum working (3 mm × 0.5 mm wire), and counter (3 cm × 0.5 mm) electrodes and a standard silver/silver chloride reference in a second compartment connected to the working cell by a capillary. A magnetic stirring bar was used to stir the contents of the cell. Additional apparatus comprised a potentiostat (Thomson Electrochem, Newcastle-Upon-Tyne) and the current flowing in the cell was monitored with a resistance box (100,000 ohms) and a chart recorder (JJ Instruments, Southampton). Prior to use the working electrode was pretreated by applying a potential sweep from −600 mV to +600 mV for 10 minutes at a sweep rate of 1 volt/second. (After each measurement the electrode was rinsed with distilled water, polished with an alumina (0.3 µm) slurry and then rinsed again). To the cell was added 1.0 ml of a biochemical "amplifier" mixture containing 0.2 mg alcohol dehydrogenase, 0.2 mg pig heart diaphorase, 10% (v/v) ethanediol, 85 mM-sodium chloride, 3% (v/v) ethanol in a 25 mM-phosphate buffer pH 7.0, followed by 1.0 ml of the pooled NADP+ solutions (containing varying amounts of NAD+) from the immunoassay. The current in the cell was measured after the addition of the ferricyanide to a final concentration of 25 mM and the application of a potential of +450 mV. The current increased linearly for the first five minutes of measurement and reached a steady value after 20 minutes. Both the rate of increase and the steady state value were proportional to the concentration of NAD+ in the sample. The rate of increase of current in µA/min was plotted against the concentration of PAP in the human serum sample and the results are shown in Figure 6. The least detectable concentration of PAP in the electrochemical immunoassay, defined as 2.5 times the standard deviation on the zero standard, was 0.24 ng/ml or 60 attomoles of antigen.

It will of course be appreciated that the nature of the antigen 3 which is determined by an electrochemical immunoassay in accordance with the present invention is irrelevant, and any desired antigen that is sufficiently large to contain several epitopes can be determined, by appropriate choice of the antibody 2 bound to the surface 1, and the antibody 4, conjugated to the enzyme label 5. In the case of a small antigen, such as progesterone, a competition reaction between antigen and enzyme labelled antigen would result in an amount of enzyme label 5 being bound by antibody 2 that is inversely proportional to the amount of antigen present. In this case the electrochemical signal would reduce as antigen concentration increases.

## Example 2

A thionine-modified graphite electrode was prepared as follows. 10 mg of thionine was dissolved in 2 ml of 90% ethanol. A 3 mm disc of graphite was immersed in the solution, and left for 10 minutes. The disc was removed, and washed thoroughly with distilled water. The disc was then washed in acetone, and finally sonicated in distilled water for 5 minutes. The graphite disc was attached to a platinum wire using a silver-loaded epoxy resin.

Electrical measurements were performed using a two-compartment glass cell that had a working volume of 2 ml. In addition to the working graphite/thionine electrode, the cell contained a 3 cm long platinum wire (0.5 mm diameter) as a counter and a saturated calomel electrode as reference. The working compartment of the cell could be stirred during operation with a magnetic stirrer bar and additions could be made directly to the cell via an injection port. Cyclic voltammograms showed a complex picture with several peaks but it was found that, at a potential of +150 mV, a satisfactory current could be obtained from a solution containing NADH. A series of measurements at this potential demonstrated that the response of the electrode was rapid and linear up to a concentration of 100 $\mu$m – NADH in a 0.1 M-sodium phosphate buffer at pH 7.0. The steady state current was reached practically instantaneously following injection of NADH and remained stable for periods in excess of 10 minutes.

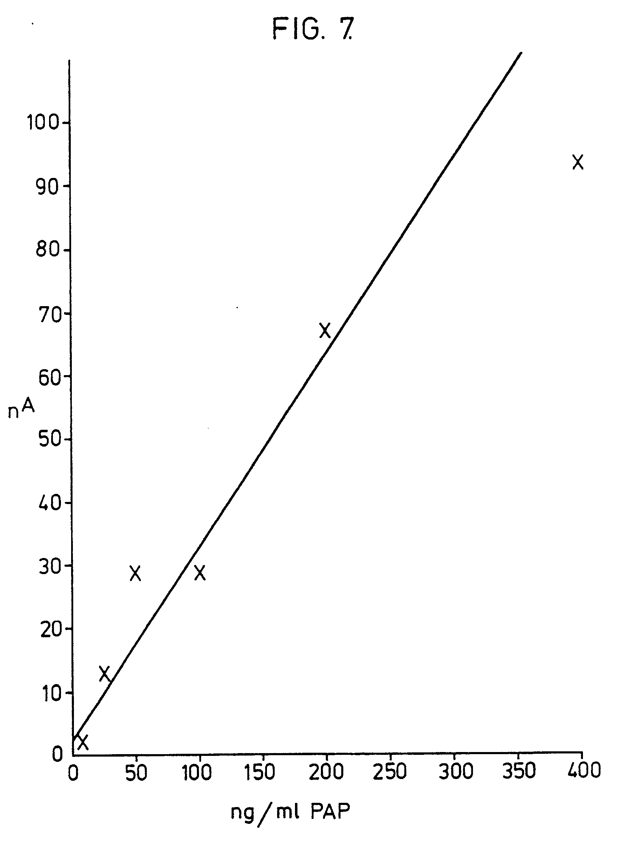

An immunoassay for PAP was then carried out in accordance with the general methods described in Example 1 using the principle described in Figure 1. The concentration of NAD$^+$ formed at each concentration formed at each concentration of PAP was measured with the thionine-modified graphite electrode. To the cell was added 1 ml of a mixture essentially the same as the biochemical amplifier described in Example 1 except that diaphorase and ferricyanide were omitted. Then, 1 ml of each pooled NADP$^+$ solution from the immunoassay was added and the steady state current was measured. The cell was washed with distilled water between measurements. The steady state current in the cell was plotted versus the concentration of PAP in the human serum sample and the results are shown in Figure 7. The least detectable concentration was 1.98 ng/ml.

## Example 3

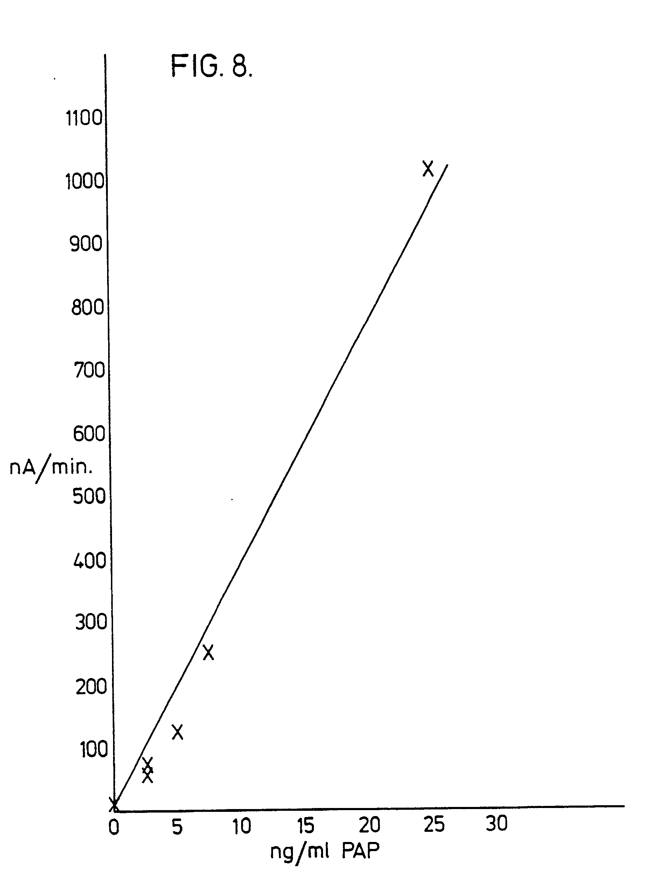

An electrochemical immunoassay for human prostatic acid phosphatase in a biochemical test cell assembly was carried out as follows, using the reaction scheme illustrated in Figure 2a. The wells of an 8 well polystyrene strip (NUNC) were coated with a monoclonal antibody to human PAP according to the procedures described in Example 1. The immunoassay was carried out as described in Example 1 up to the addition of 100 $\mu$l of the NADP$^+$ solution to the wells. After 20 minutes of incubation (at 22°) with this substrate, 100 $\mu$l of a mixture which was essentially the same as the biochemical amplifier described in Example 1, but which also contained ferricyanide at 25 mM was added to each well. The phosphate buffer in the biochemical amplifier inhibited the further reaction of the enzyme label, Alkaline phosphatase, and the NAD$^+$ formed in each well was able to trigger the biochemical amplifier. Immediately after addition of the biochemical amplifier to each well a lid incorporating a pair for each well, of platinum working and counter electrodes (each 3 mm × 0.5 mm) was placed over the 8 well strip. The platinum electrodes were fully immersed in the solution in each well and were cemented to insulated copper pins 2 mm in diameter using silver-loaded epoxy resin. The copper pins were pushed through holes drilled in a polystyrene sheet of dimensions 8 cm × 1 cm × 3 mm to form the lid and each pin was connected to a software driven switch as shown in Figure 5. Prior to use the platinum working electrodes were pretreated by a potential excursion from –600 mV to +600 mV in distilled water and each working electrode had been polished with an alumina slurry (0.3 $\mu$m). The test well assembly and lid was then taped to the top plate of a Titertek plate shaker (Flow Laboratories) and vigorously agitated to simulate a stirred cell. A potential of +450 mV was applied to each working electrode in turn via the user port of a BBC microcomputer (with appropriate software for poising the potential and recording current from two electrode systems). The rate of increase of the current in each well was measured for two minutes by the microcomputer. The rate of increase in the current ((nA/min) was plotted against the concentration of PAP in the human serum sample and the results are shown in Figure 8.

In this example the activity of the enzyme label in the immunoassay was stopped before the addition of the biochemical amplifier. The alternative of allowing the activity to continue was not used owing to the more complex kinetics of current generation caused by the continuous appearance of the trigger molecule NAD$^+$ throughout the measurement period.

## Claims

1. A method of determining a biochemical substance in a sample, which method comprises carrying out an assay so as to biochemically bind to a solid support an amount of an enzyme dependent upon the amount of the said substance present in the sample, and determining the amount of enzyme bound to the solid support electrochemically by measuring the electrical change produced by a redox reaction at an electrode, characterised in that the bound enzyme does not itself catalyse a redox reaction, but catalyses a reaction which results in the production of a trigger substance for a cyclic chemical reaction or series of reactions, at least one product of which is the said trigger substance and at least one further product of which is capable of causing a redox reaction to take place at the electrode, which can be determined electrochemically.

2. A method as claimed in Claim 1, wherein the trigger substance is a nicotinamide adenine dinucleotide co-enzyme.

3. A method as claimed in Claim 1 or Claim 2, wherein the said further product is lipoamide dehydrogenase.

4. A method as claimed in Claim 1 or Claim 2, wherein one of the cyclic series of reactions is a dehydrogenation reaction which is conducted in the presence of an NAD+-specific dehydrogenase.

5. A method as claimed in any one of the preceding claims wherein a biochemical ligand is bound to a surface of the electrode, and the ligand reacts with a corresponding antiligand during the immunoassay.

6. A method as claimed in any one of the preceding claims, wherein the said enzyme is a phosphatase or a hydrolase.

7. A method as claimed in any one of the preceding claims, wherein the cyclic chemical reaction is an NAD+/NADH cycle, a catechol/quinone cycle, a thiol/disulphide cycle, an FAD/FADH2 cycle or a PGG/PQQH2 cycle.

8. A method as claimed in Claim 6 and Claim 7, wherein the enzyme is alkaline phosphatase, the cycle chemical reaction is an NAD+/NADH cycle, and the trigger substance is NAD+.

9. A method as claimed in any one of the preceding claims, wherein the reaction mixture comprises at least one electron transfer mediator which can transfer electrons between the said further product and the electrode.

10. A method as claimed in Claim 9, wherein the electron transfer mediator is ferrocene or a derivative thereof and/or ferricyanide.

11. A method as claimed in any one of the preceding Claims, wherein the determination is carried out in a biochemical test cell assembly, comprising at least one test cell for containing a test solution, a pair of electrodes in the test cell, and a biochemical ligand bound to a surface in the cell, the said ligand being adapted to bind specifically with a corresponding antiligand, thereby to immobilise on the said surface an enzyme capable of being detected, indirectly, by its ability to trigger a further chemical reaction employing a redox cycle with an electrical effect on the said electrodes.

12. A method as claimed in Claim 11, wherein the assembly comprises a plurality of test cells each containing a pair of electrodes, the electrodes of the test cells each being connected to a track of a printed circuit board forming a part of the assembly.

13. A method as claimed in Claim 12, wherein the assembly includes a multi-way connector to enable the simultaneous connection of measuring equipment to a plurality of pairs of electrodes in the assembly.

14. A method as claimed in any one of Claims 11 to 13, wherein the assembly includes means to read sequently electrical values associated with a plurality of electrode pairs.

15. A method as claimed in Claim 14, wherein the means for reading comprises a computer adapted to read repeatedly a sequence of values from the said plurality of electrode pairs, and store the values associated with each reading, so as to provide a profile of the change in the said electrical value for each of the electrode pairs, over a period of time.

16. A method as claimed in any one of claims 13 to 15, wherein the assembly comprises at least eight test cells, each having a pair of electrodes.

17. A method as claimed in any one of claims 12 to 16, wherein at least one electrode of the or each pair is attached to a removable cover portion for the respective test cell.

18. A method as claimed in any one of claims 12 to 17, wherein the said surface to which the ligand is bound is a surface of one of the said electrodes.

## Revendications

1. Procédé de détermination d'une substance biochimique dans un échantillon, procédé qui consiste à effectuer un dosage de façon à fixer biochimiquement sur un support solide une quantité d'une enzyme dépendant de la quantité de ladite substance présente dans l'échantillon, et à déterminer la quantité d'enzyme liée au support solide par voie électrochimique en mesurant le changement électrique produit par une réaction redox à une électrode, caractérisé en ce que l'enzyme liée ne catalyse pas elle-même une réaction redox, mais catalyse une réaction qui entraîne la production d'une substance de déclenchement pour une réaction chimique cyclique ou une série de réactions chimiques cycliques, au moins un produit de laquelle est ladite substance de déclenchement et au moins un autre produit de laquelle peut faire qu'ait lieu à l'électrode une réaction redox qui peut être déterminée par voie électrochimique.

2. Procédé selon la revendication 1, dans lequel la substance de déclenchement est un co-enzyme nicotinamide adénine dinucléotide.

3. Procédé selon la revendication 1 ou la revendication 2, dans lequel ledit autre produit est une lipoamide déshydrogénase.

4. Procédé selon la revendication 1 ou la revendication 2, dans lequel une réaction de la série cyclique de réactions est une réaction de déshydrogénation qui est effectuée en présence d'une déshydrogénase spécifique de NAD⁺.

5. Procédé selon l'une quelconque des revendications précédentes, dans lequel un ligand biochimique est lié à une surface de l'électrode, et le ligand réagit avec un antiligand correspondant pendant l'immunodosage.

6. Procédé selon l'une quelconque des revendications 1 à 5, dans lequel ladite enzyme est une phosphatase ou une hydrolase.

7. Procédé selon l'une quelconque des revendications précédentes, dans lequel la réaction chimique cyclique est un cycle NAD⁺/NADH, un cycle catéchol/quinone, un cycle thiol/disulfure, un cycle FAD/FADH2 ou un cycle PGG/PQQH2.

8. Procédé selon la revendication 6 et la revendication 7, dans lequel l'enzyme est la phosphatase alcaline, la réaction chimique cyclique est un cycle NAD⁺/NADH et la substance de déclenchement est NAD⁺.

9. Procédé selon l'une quelconque des revendications 1 à 8, dans lequel le mélange réactionnel comprend au moins un médiateur de transfert d'électrons qui peut transférer des électrons entre ledit autre produit et l'électrode.

10. Procédé selon la revendication 9, dans lequel le médiateur de transfert d'électrons est le ferrocène ou un de ses dérivés et/ou un ferricyanure.

11. Procédé selon l'une quelconque des revendications précédentes, dans lequel la détermination est effectuée dans un ensemble de cellules d'essai biochimique, comprenant au moins une cellule d'essai destinée à contenir une solution d'essai, une paire d'électrodes dans la cellule d'essai, et un ligand biochimique fixé à une surface dans la cellule, ledit ligand étant adapté pour fixer sélectivement un antiligand correspondant, en immobilisant ainsi sur ladite surface une enzyme pouvant être détectée, indirectement, par son aptitude à déclencher une autre réaction chimique utilisant un cycle redox avec effet électrique sur lesdites électrodes.

12. Procédé selon la revendication 11, dans lequel l'ensemble comprend plusieurs cellules d'essai contenant chacune une paire d'électrodes, chaque électrode des cellules d'essai étant reliée à une piste d'une plaquette à circuits imprimés formant partie de l'ensemble.

13. Procédé selon la revendication 12, dans lequel l'ensemble comprend un connecteur multiple pour permettre la connexion simultanée d'un équimement de mesure à plusieurs paires d'électrodes de l'ensemble.

14. Procédé selon l'une quelconque des revendications 11 à 13, dans lequel l'ensemble comprend des moyens pour lire successivement les valeurs électriques associées à plusieurs paires d'électrodes.

15. Procédé selon la revendication 14, dans lequel le moyen de lecture comprend un ordinateur conçu pour lire de façon répétée une séquence de valeurs à partir des plusieurs paires d'électrodes, et à stocker les valeurs associées à chaque lecture, de façon à fournir un profil du changement de ladite valeur électrique pour chacune des paires d'électrodes, sur une certaine période de temps.

16. Procédé selon l'une quelconque des revendications 13 à 15, dans lequel l'ensemble comprend au moins huit cellules d'essai, chacune comportant une paire d'électrodes.

17. Procédé selon l'une quelconque des revendications 12 à 16, dans lequel au moins une électrode de la paire ou de chaque paire est fixée à une portion formant couvercle amovible pour la cellule d'essai correspondante.

18. Procédé selon l'une quelconque des revendications 12 à 17, dans lequel ladite surface à laquelle est lié le ligand est une surface de l'une desdites électrodes.

## Ansprüche

1. Verfahren zur Messung einer biochemischen Substanz in einer Probe, bei dem man einen Test durchführt, um biochemisch an einen festen Träger eine Menge eines Enzyms in Abhängigkeit der Menge der in der Probe vorhandenen Substanz zu binden und die Menge des an den festen Träger gebundenen Enzyms elektrochemisch bestimmt, indem man die elektrische Änderung mißt, die durch eine Redoxreaktion an einer Elektrode erzeugt wird, **dadurch gekennzeichnet,** daß das gebundene Enzym selbst keine Redoxreaktion katalysiert, jedoch eine Reaktion katalysiert, die zur Produktion einer Triggersubstanz für eine zyklische chemische Reaktion oder Serien von Reaktionen führt, wobei mindestens ein Produkt davon die Triggersubstanz ist und mindestens ein weiteres Produkt davon in der Lage ist, eine Redoxreaktion an der Elektrode zu bewirken, die elektrochemisch bestimmt werden kann.

2. Verfahren nach Anspruch 1, bei dem die Triggersubstanz ein Nikotinamidadenin-Dinukleotid-Coenzym ist.

3. Verfahren nach Anspruch 1 oder 2, bei dem das weitere Produkt Lipoamiddehydrogenase ist.

4. Verfahren nach Anspruch 1 oder 2, bei dem eine der zyklischen Reihen von Reaktionen eine Dehydrogenierungsreaktion ist, die in Gegenwart von einer NAD⁺-spezifischen Dehydrogenase durchgeführt wird.

5. Verfahren nach einem der vorhergehenden Ansprüche, bei dem ein biochemischer Ligand an die Oberfläche der Elektrode gebunden wird und der Ligand mit einem entsprechenden Antiligand während des Immunoassays reagiert.

6. Verfahren nach einem der vorhergehenden Ansprüche, bei dem das Enzym eine Phosphatase oder eine Hydrolase ist.

7. Verfahren nach einem der vorhergehenden Ansprüche, bei dem die zyklische chemische Reaktion ein NAD⁺/NADH-Zyklus, ein Katechol/Chinon-Zyklus, ein Thiol/DisulfidZyklus, ein FAD/FADH2-Zyklus, oder ein PGG/PQQH2-Zyklus ist.

8. Verfahren nach Anspruch 6 und 7, bei dem das Enzym alkalischer Phosphatase, die zyklische chemische Reaktion ein NAD⁺/NADH-Zyklus, und die Triggersubstanz NAD⁺ ist.

9. Verfahren nach einem der vorhergehenden Ansprüche, bei dem die Reaktionsmischung mindestens einen Elektronentransfermediator enthält, der Elektronen zwischen dem weiteren Produkt und der Elektrode übertragen kann.

10. Verfahren nach Anspruch 9, bei dem der Elektronentransfermediator Ferrocen oder ein Derivat davon und/oder Ferricyanid ist.

11. Verfahren nach einem der vorhergehenden Ansprüche, bei dem die Bestimmung in einer biochemischen Testzellvorrichtung durchgeführt wird, die mindestens eine Testzelle zur Aufnahme der Testlösung, ein Paar Elektroden in der Testzelle und einen biochemischen Ligand, gebunden an die Oberfläche der Zelle, umfaßt, wobei der Ligand angepaßt wird, um spezifisch mit einem entsprechenden Antiligand zu reagieren, um dadurch auf der Oberfläche ein Enzym zu immobilisieren, das indirekt durch seine Fähigkeit eine weitere chemische Reaktion unter Verwendung eines Redoxzyklus mit einem elektrischen Effekt auf den Elektroden zu triggern, nachgewiesen werden kann.

12. Verfahren nach Anspruch 11, bei dem der Aufbau eine Vielzahl von Testzellen umfaßt, wobei jede ein Paar Elektroden enthält, wobei die Elektroden der Testzellen jeweils mit einer Leiterbahn einer gedruckten Schaltung verbunden sind, die einen Teil des Aufbaus bildet.

13. Verfahren nach Anspruch 12, bei dem der Aufbau eine Vielwegverbindung enthält, um die gleichzeitige Verbindung des Meßsystems mit einer Vielzahl von Elektrodenpaaren im Aufbau zu ermöglichen.

14. Verfahren nach einem der Ansprüche 11 bis 13, bei dem der Aufbau Mittel umfaßt, um regelmäßig elektrische Werte, die mit einer Vielzahl der Elektrodenpaare in Verbindung stehen, aufzuzeichnen.

15. Verfahren nach Anspruch 14, bei dem die Mittel zum Aufzeichnen einen Computer umfassen, der zur wiederholten Aufzeichnung einer Reihenfolge von Werten aus der Vielzahl von Elektrodenpaaren und zur Speicherung der Werte, die mit jeder Aufzeichnung verbunden sind, adaptiert ist, um so ein Profil der Änderung in dem elektrischen Wert für jedes der Elektrodenpaare über einen Zeitraum zur Verfügung zu stellen.

16. Verfahren nach einem der Ansprüche 13 bis 15, bei dem der Aufbau mindestens acht Testzellen umfaßt, wobei jede ein Paar Elektroden aufweist.

17. Verfahren nach einem der Ansprüche 12 bis 16, bei dem mindestens eine Elektrode von jedem Paar oder jedes Paares an einen entfernbaren Abdeckteil für die jeweilige Testzelle angebunden ist.

18. Verfahren nach einem der Ansprüche 12 bis 17, bei dem die Oberfläche, an die der Ligand gebunden wird, eine Oberfläche von einer der Elektroden ist.

FIG. 1.

FIG. 2a.

FIG. 2b.

FIG.2c.

FIG. 2d.

FIG.2e.

# FIG. 3.

# FIG. 5.

FIG. 4.

FIG. 6.

FIG. 7

# FIG. 8.